# EUROPEAN PATENT SPECIFICATION

(11) **EP 3 285 819 B1**
(45) Date of publication and mention of the grant of the patent: **04.11.2020**
(21) Application number: 15889426.1
(22) Date of filing: 10.09.2015
(51) Int. Cl.: A61L 27/40, A61L 27/24, A61L 27/56

(54) **CORNEA MIMETIC BIOMATERIALS: VITRIFIED COLLAGEN-CYCLODEXTRIN IMPLANTS**
MIMETISCHE HORNHAUTBIOMATERIALIEN: KERAMISCHE KOLLAGEN-CYCLODEXTRINIMPLANTATE
BIOMATÉRIAUX MIMÉTIQUES POUR LA CORNÉE : IMPLANTS DE CYCLODEXTRINE-COLLAGÈNE VITRIFIÉS

(30) Priority: 24.04.2015 WO PCT/US2015/027503; 04.05.2015 US 201562156833 P
(43) Date of publication of application: 28.02.2018
(73) Proprietor: The Johns Hopkins University, Baltimore, MD 21218 (US)
(72) Inventor: ELISSEEFF, Jennifer H., Baltimore, Maryland 21213 (US); GUO, Qiongyu, Rockville, MD 20852-1183 (US); MAJUMDAR, Shoumyo, Baltimore, Maryland 21218 (US); SINGH, Anirudha, Baltimore, Maryland 21209 (US)
(74) Representative: Murgitroyd & Company
(86) International application number: PCT/US2015/049371
(87) International publication number: WO 2016/171745

(56) References cited:
- WO-A1-96/31220
- WO-A1-2015/164733
- US-A1- 2004 115 271
- US-A1- 2010 221 319
- GUO QIONGYU ET AL: "Developing biomimetic collagen-based matrix using cyclodextrin for corneal repair", 2014 40TH ANNUAL NORTHEAST BIOENGINEERING CONFERENCE (NEBEC), IEEE, 25 April 2014 (2014-04-25), pages 1-2, XP032692798, DOI: 10.1109/NEBEC.2014.6972807 [retrieved on 2014-12-02]
- GUO, Q. ET AL.: 'Regulation of Collagen Fibrillogenesis and Alignment Using Cyclodextrin for Corneal Repair' INVESTIGATIVE OPHTHALMOLOGY & VISUAL SCIENCE vol. 55, no. ISSUE, April 2014, pages 13 - 6444, XP008183017
- CHELLAM, J. ET AL.: 'Influence of Cyclodextrins on the Physical Properties of Collagen' INTERNATIONAL JOURNAL OF PHARMA AND BIO SCIENCE October 2013, ISSN 0975-6299 pages B-795 - B-806, XP055324063

## Description

### BACKGROUND OF THE INVENTION

Due to keratitis, keratoconus, other diseases and injuries, a large population in the world today suffers from corneal blindness. However, due to the limited availability of donor corneas especially in developing regions of Asia and Africa, not all individuals can receive treatment.

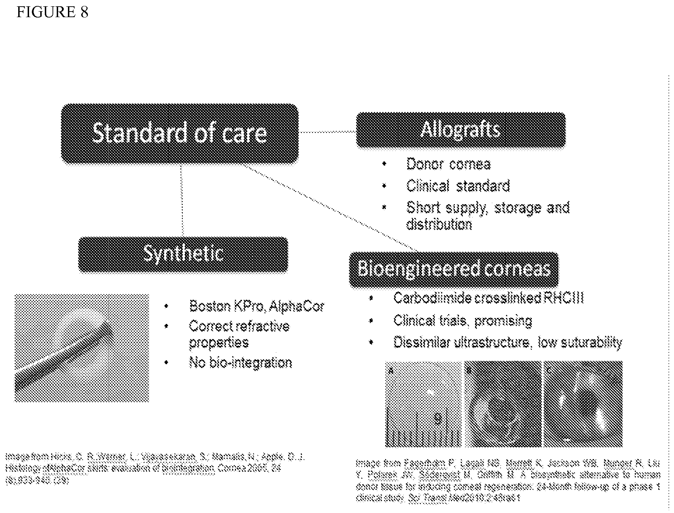

The following is a briefing of current standards. Existing standards of care include donor corneas, the best cornea replacement currently available. However, there are issues of availability, storage and distribution. Synthetic corneas, or keratoprosthesis such as the Boston Kpro, or AlphaCor also exist in the clinic. However, while they can successfully correct for refractive properties of the damaged cornea, they are synthetic polymer based, and do not support tissue remodeling or biointegration, leading to complications. There are bioengineered corneas in clinical trials - such as the carbodiimide crosslinked RHCIII. These show great promise. However, these materials have low suturability and a collagen organizational ultrastructure that does not match that of the cornea (Fig. 8).

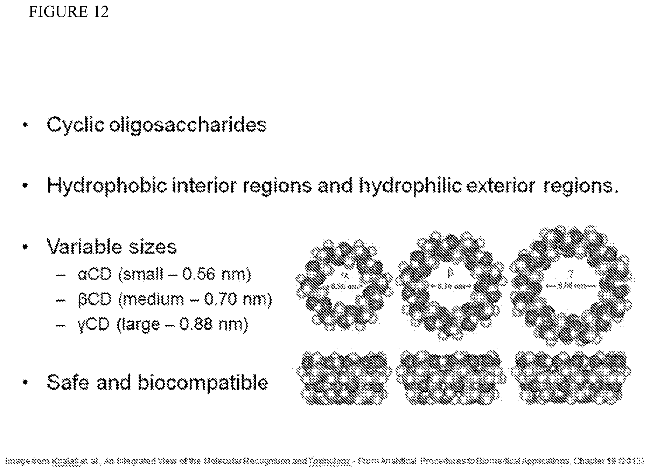

In the normal collagen assembly seen in the cornea there are a large number of proteoglycans such as decorin and lumican the assist with the triple helix formation, fibril alignment and organization. We wanted to replicate this in the laboratory setting using molecules that can behave as artificial chaperones and can provide these same functions. The inventors' early focus was on cyclodextrins as such molecules (Fig. 12) - small cyclic oligosaccharides classified depending on the number of glucose monomers in the ring which were detailed in WO 2015/164733. These have been previously used in cancer therapy and other applications for drug delivery and have been deemed very safe and biocompatible.

Cyclodextrins (CDs) are a family of cyclic oligomers composed of a ring of six to eight glucose molecules. These ring molecules feature an inner hydrophobic core and an outer hydrophilic ring that can form complexes with small molecules or portions of large compounds. The solubility of natural cyclodextrins is very poor and initially this prevented cyclodextrins from becoming effective complexing agents. In the late 1960's, it was discovered that chemical substitutions at the 2-, 3-, and 6-hydroxyl sites would greatly increase solubility. The degree of chemical substitution and the nature of the groups used for substitution determine the final maximum concentration of cyclodextrin in an aqueous medium. Most chemically modified cyclodextrins are able to achieve a 50% (w/v) concentration in water. In addition, substitutions and reactions at the hydroxyl groups can provide additional functionality, by, for example, converting the groups to acids, amines, or others.

Extracellular matrix (ECM) is a complex mixture of macromolecules consisting of proteins, proteoglycans, and other soluble molecules. Collagen, the most abundant protein in animals, is widely applied in various bioapplications. However, little effort has been made on engineering an ECM scaffold composed of both collagen and proteoglycans due to difficulty of deriving large amount of purified proteoglycans. In native cornea, the proteoglycans play a critical role in corneal transparency by regulating the collagen fibril diameter and spacing. Therefore, there still exists a need for proteoglycan substitutes to develop biomimetic collagen-based ECM to solve the challenging issues associated with clinical applications, such as corneal regeneration.

Guo, Q. et al., "Developing biomimetic collagen-based matrix using cyclodextrin for corneal repair", 2014 40th Annual Northeast Bioengineering Conference, IEEE, 25 April 2014, pages 1-2, discloses the use of cyclodextrins (CDs) (α-CD, β-CD and/or γ-CD) as a proteoglycan substitute to engineer a bimimetic collagen-based matrix. The use of cyclodextrins increased collagen thermal stability and reduced collagen fibrogenesis. As a result, a thick transparent and mechanically strong collagen-based membrane was formed, which the authors considered to have potential to be used as a therapeutic eye patch for corneal repair. The membranes were formed by a three-stage sequence of gelation, vitrification and rehydration. Guo, Q. et al., "Regulation of collagen fibrillogenesis and alignment using cyclodextrin for corneal repair", Investigative Ophthalmology & Visual Science, Vol. 55, April 2014, discloses Type I collagen-based membranes incorporated with different cyclodextrins (α-CD, β-CD and/or γ-CD) prepared following a three-stage sequence of gelation, vitrification and rehydration. The authors disclosed that Type I collagen-CD membranes were developed with optimized optical and mechanical properties for corneal regeneration.

### SUMMARY OF THE INVENTION

In accordance with the disclosed embodiments, the present invention provides compositions for preparing a true corneal mimetic structure. The cornea is a transparent multilayered tissue consisting of epithelial and endothelial layers, with the thickest region being the corneal stroma, largely a collagen I based extracellular matrix, sparsely populated with keratocytes. In some embodiments, one possible strategy is to develop a corneal biomimetic which focuses on replicating the stroma, following which, other layers can be added. This replicated stroma is then used as a base to fabricate collagen based corneal implants which can be tuned to self-assemble into cornea mimetic structures. The goal is to mimic the ultrastructure and physical properties of the native healthy cornea.

In accordance with an embodiment, the present invention provides a composition or membrane that comprises aligned fibrils comprising collagen and cyclodextrin as claimed in claim 1. The composition or membrane comprises aligned fibrils of a vitrified matrix gel comprising collagen and cyclodextrin.

Aligned fibrils are distinguished from a more random orientation and can be assessed by methods such as transmission electron microscopy evaluation of a sample to detect an aligned orientation rather than a more random configuration.

In the present compositions or membranes, various types of collagen may be suitably employed, including Type I, Type II, Type III and Type IV collagen may be employed. For certain compositions or membranes, Type I collagen can be employed. In certain compositions or membranes, it may be preferred that a substantial portion of the collagen content of the composition is Type I collagen, for example where 40, 50, 60, 70, 8, 90, 95 or 100 weight percent of the total collagen content in the composition or membrane is Type I collagen.

Various cyclodextrins also may be employed and the composition comprises α-cyclodextrin (α-CD), β-cyclodextrin (β-CD) and/or γ-cyclodextrin (γ-CD). The compositions according to the claims comprise α-CD, β-CD and/or y-CD comprising a plurality of hydroxyl groups chemically substituted with a different functional group or moiety wherein the different functional group or moiety is a hydrophilic group. For certain compositions or membranes, α-CD may be preferred, including in combination with Type I collagen. In certain compositions or membranes, it may be preferred that a substantial portion of the cyclodextrin content of the composition or membrane is α-CD, for example where 40, 50, 60, 70, 8, 90, 95 or 100 weight percent of the total cyclodextrin content in the composition or membrane is α-cyclodextrin (α-CD), including in combination with Type I collagen.

The present compositions or membranes also may comprise one or more additional biologically active or therapeutic agents, such as for example indomethacin.

The present compositions or membranes are especially useful to serve as a drug reservoir to extend the release of drugs to a patient.

The present compositions or membranes are multilayered. The composition or membrane may be formed into a variety of configurations including a shape suitable for use as an artificial cornea, and/or a shape suitable for use as a corneal replacement, patch or graft. The present compositions and membranes will have additional uses as a variety of other biological materials.

The inventors hypothesized that, similar to proteoglycan in native tissues, the incorporation of cyclodextrins in collagen matrix would regulate collagen fibrogenesis while preserving collagen triple helical formation. Traditional engineered type I collagen matrices with fibrillar architectures are opaque, whereas transparent gels composed of amorphous collagen networks exhibit poor mechanical properties. It was expected that cyclodextrins could be added to a collagen matrix as a proteoglycan substitute and would assist in optimizing both optical and mechanical properties for corneal regeneration.

In accordance with an embodiment, the present invention provides a composition as claimed in claim 1 comprising a vitrified matrix gel having a first component and a second component, wherein the first component comprises collagen, and wherein the second component comprises cyclodextrin.

In accordance with another embodiment, the present invention provides a composition as claimed in claim 1 comprising a vitrified matrix gel having a first component and a second component, wherein the first component comprises collagen, and wherein the second component comprises cyclodextrin, and further comprises at least one biologically active agent.

Disclosed, but not claimed, is a method for making a vitrified matrix gel having a first component and a second component, wherein the first component comprises collagen, and wherein the second component comprises cyclodextrin, comprising: a) obtaining an aqueous solution of collagen; b) obtaining an aqueous solution of cyclodextrin; c) combining the solutions of a) and b); and d) dehydrating the combined solution of c) for a period of time sufficient to allow vitrification of the solution.

In accordance with a yet another embodiment, the present invention provides the compositions described above for use as a matrix for repair of a tissue of a mammal.

In accordance with another embodiment, the present invention provides the compositions described above for use as a matrix for repair of the cornea of an eye of a mammal.

In accordance with further examples of the disclosure, there are provided ocular compositions that can be fabricated in layers, with different functionalities per layer to better mimic the cornea.

### BRIEF DESCRIPTION OF THE DRAWINGS

Figure 1A depicts differential scanning calorimetry (DSC) first heating curve of three collagen-based membranes, including normal collagen vitrigel (CV), α-CD-col composition of the present invention, and crosslinked CV.
Figure 1B shows that α-CDs induced various collagen ultrastructures (cross-sectional views).
Figure 2 is a graph showing that the α-CD-col composition of the present invention exhibited an excellent transparency with a transmittance of as high as 96% at 550 nm. An inset in the figure shows a photograph of a wet membrane placed on a printed word "eye".
Figure 3 is a graph depicting Load vs. displacement of a suturablity test of two α-CD-col compositions of the present invention with a thickness of 520 µm and 170 µm, respectively. An inset in the figure shows a photograph of the thicker membrane after stretching over 5.6 mm.
Figure 4 is a photograph of an apparatus used in the load test of the compositions of the present invention.
Figure 5 shows a schematic of cell protrusion analysis and the effect of different vitrigel composition collagen density of primary cultures of bovine keratocytes.
Figure 6 depicts how gene expression of three different gene markers in primary cultures of bovine keratocytes is affected by the fibril nanoarchitecture of the vitrigel compositions of the present invention.
Figure 7 is a schematic showing the architecture of various cyclodextrins and how they interact with collagen fibrils in solution. The spaces in the cyclodextrin molecules can be used as drug reservoirs for water insoluble drugs and biologically active agents.
Figure 8 is an illustration depicting the standard of care options for corneal repairs and grafts.
Figure 9 is an illustration depicting the development of one or more embodiments of the corneal mimetic biomaterials of the present invention. The illustration depicts how the collagen based biomaterials of the present invention mimic the ultrastructure and physical properties of the native healthy cornea.
Figure 10 is an image from Biomaterials 34:9365-9372 (2013) showing an illustration of how vitrification leads to formation of collagen vitrigels with high collagen density, superior strength, and transparency.
Figures 11a-11b are an illustration depicting the contrast between prior art methods for collagen assembly and one or more examples of the present disclosure. 11a) In vivo self-assembly of collagen type I into ordered fibrillar ultrastructure involves small proteoglycans such as decorin. 11b) In vitro, similar ultrasctructure can be obtained using cyclodextrins as molecular chaperones for collagen triple helices.
Figure 12 is a prior art image depicting cyclodextrin structures. Cyclodextrins are composed of 5 or more α-D-glucopyranoside units linked 1->4, as in amylose (a fragment of starch). The 5-membered macrocycle is not natural. Recently, the largest well-characterized cyclodextrin contains 32 1,4-anhydroglucopyranoside units, while as a poorly characterized mixture, at least 150-membered cyclic oligosaccharides are also known. Typical cyclodextrins contain a number of glucose monomers ranging from six to eight units in a ring, creating a cone shape.
Figure 13 shows the transparency of the CD Col implant embodiments of the present invention.
Figure 14 depicts the cornea mimetic ultrastructure of the CD Col implant embodiments of the present invention.
Figure 15 shows a close up photomicrograph of the biomimetic ultrastructure of βCD-COOH Col implants of the present invention.
Figure 16 depicts photomicrographs of keratocytes and epithelial cells showing the biocompatibility of the implant compositions of the present invention.
Figure 17 depicts H & E stained sections showing biocompatibility of the implant compositions of the present invention.
Figure 18 depicts photographs of in vivo corneal implantation of a βCD CV implant of the present invention.

### DETAILED DESCRIPTION OF THE INVENTION

In accordance with one or more embodiments, the present inventors employed cyclodextrins for use as a proteoglycan substitute to engineer a biomimetic collagen-based matrix composition. The resulting incorporation of cyclodextrin in the inventive collagen compositions increased collagen thermal stability and reduced collagen fibrogenesis. As a result, a thick, transparent and mechanically strong collagen-based composition was formed. These cyclodextrin-collagen compositions hold great potential to be used as a therapeutic eye implants for corneal repair.

In accordance with an embodiment, the present invention provides a composition as claimed in claim 1 comprising a vitrified matrix gel having a first component and a second component, wherein the first component comprises collagen, and wherein the second component comprises cyclodextrin.

The collagen-cyclodextrin composition is fashioned into a multilayered or multilamellar structure, where thickness of each layer can be controlled. For example, the human cornea is 10-500 micrometers thick. The present invention can be made to thicknesses (micrometers) of 10-500, 25-500; 50-500; 100-500; 250-500. The multilayer structure and support multi-functionality, and thus the composition functions more similarly to the cornea than current artificial corneas, grafts or patches. Additionally, the compositions can be coated at the surface to enhance biological action.

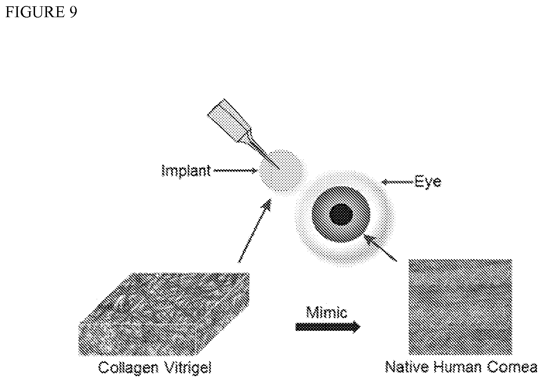

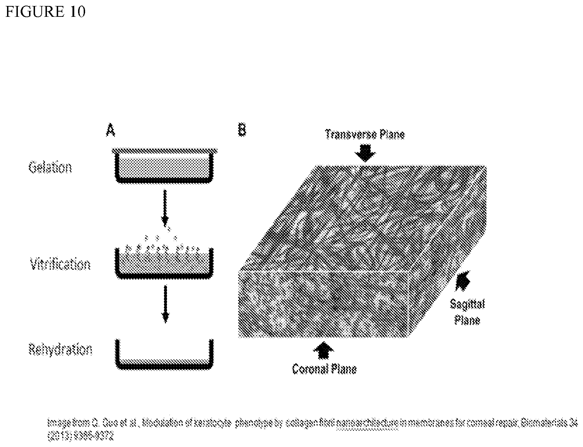

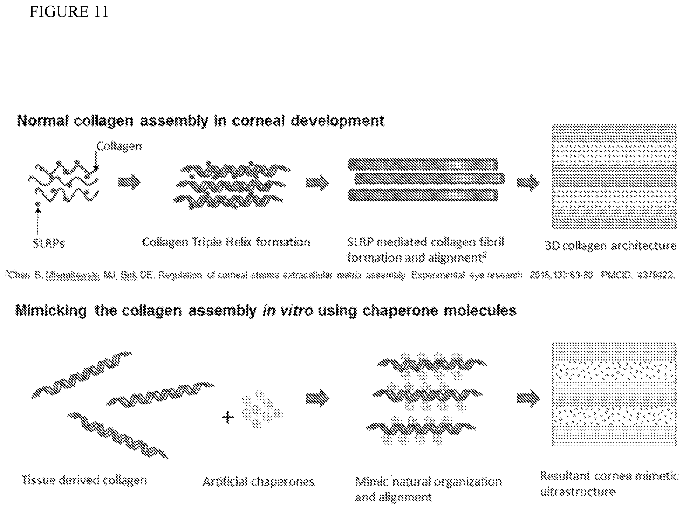

In accordance with one or more embodiments of the compositions of the present invention, the inventors used collagen vitrigel technology. Collagen gel vitrification is a slow dehydration process at controlled temperature and controlled humidity that results in formation of collagen membranes or sheets of high transparency and strength. The release of bound water from the collagen results in an increase in entropy driving the formation a high density of fibrils. Vitrification of collagen gels allows the formation of vitrigel membranes with high collagen density, superior strength and higher transparency (Three steps: Gelation, Vitrification, Rehydration). Previously, CV membranes with higher thicknesses (500 um) had very low transparency (See, Figs. 9, 10, and 11).

As used herein, the term "vitrification" or "vitrigel" means that the composition is composed of an aqueous solution of a mixture of one or more collagens and one or more cyclodextrins and allowed to form a hydrogel. In some embodiments, the gelation of the composition is performed at a temperature of 37 °C. After the hydrogel is formed, the hydrogel is vitrified by dehydration, such as, for example, heating the hydrogel at a specific temperature and humidity, for a specific length of time to allow vitrification to occur. In some embodiments, the vitrification is performed at a temperature of 35 to 45 °C and a humidity of between about 30% and 50% relative humidity. In an embodiment, the vitrification is performed at a temperature of 40 °C and a relative humidity of 40%. The time needed for vitrification of the compositions can vary from a few days to a few weeks. In an embodiment, the time for vitrification of the compositions is about 1 to 2 weeks.

"Gel" refers to a state of matter between liquid and solid, and is generally defined as a cross-linked polymer network swollen in a liquid medium. Typically, a gel is a two-phase colloidal dispersion containing both solid and liquid, wherein the amount of solid is greater than that in the two-phase colloidal dispersion referred to as a "sol." As such, a "gel" has some of the properties of a liquid (i.e., the shape is resilient and deformable) and some of the properties of a solid (i.e., the shape is discrete enough to maintain three dimensions on a two-dimensional surface).

By "hydrogel" is meant a water-swellable polymeric matrix that can absorb water to form elastic gels, wherein "matrices" are three-dimensional networks of macromolecules held together by covalent or noncovalent crosslinks. On placement in an aqueous environment, dry hydrogels swell by the acquisition of liquid therein to the extent allowed by the degree of cross-linking.

The compositions of the present invention comprise collagen. The collagen of the first component is selected from the group consisting of Type I, Type II, Type III and Type IV collagen. In an embodiment, the collagen used as the first component of the composition is Type I collagen. One of ordinary skill in the art would understand that the collagen used in the compositions and methods could include more than one type of collagen.

The compositions of the present invention also comprise cyclodextrins. The cyclodextrin of the second component is selected from the group consisting of α-cyclodextrin, β-cyclodextrin, and γ-cyclodextrin. In an embodiment, the cyclodextrin used as the second component of the composition is α-cyclodextrin. One of ordinary skill in the art would understand that the cyclodextrin used in the compositions and methods could include more than one type of cyclodextrin.

The cyclodextrins used in the inventive compositions have a plurality of hydroxyl groups chemically substituted with another functional group or moiety wherein the different functional group or moiety is a hydrophilic group.

As used herein, the vitrified compositions of the present invention are hydrated prior to use.

The vitrigel compositions of the present invention are optically transparent and suitable for a variety of uses. In one embodiment the vitrigel composition has an optical transparency of about 96% at 550 nm.

It will be understood that the vitrigel compositions of the present invention can be molded or formed into any particular shape suitable for use as a replacement tissue or tissue filler. The instant invention provides for ex vivo polymerization techniques to form scaffolds and so on that can be molded to take the desired shape of a tissue defect, promote tissue development by stimulating native cell repair, and can be potentially implanted by minimally invasive injection.

In one or more embodiments, the vitrigel compositions of the present invention can be shaped for use as an artificial cornea for a subject.

In accordance with another embodiment, the present invention provides a composition as claimed in claim 1 comprising a vitrified matrix gel having a first component and a second component, wherein the first component comprises collagen, and wherein the second component comprises cyclodextrin, and further comprises at least one biologically active agent.

An "active agent" and a "biologically active agent" are used interchangeably herein to refer to a chemical or biological compound that induces a desired pharmacological and/or physiological effect, wherein the effect may be prophylactic or therapeutic. The terms also encompass pharmaceutically acceptable, pharmacologically active derivatives of those active agents specifically mentioned herein, including, but not limited to, salts, esters, amides, prodrugs, active metabolites, analogs and the like. When the terms "active agent," "pharmacologically active agent" and "drug" are used, then, it is to be understood that the invention includes the active agent per se as well as pharmaceutically acceptable, pharmacologically active salts, esters, amides, prodrugs, metabolites, analogs etc.

Incorporated," "encapsulated," and "entrapped" are art-recognized when used in reference to a therapeutic agent, dye, or other material and a polymeric composition, such as a composition of the present invention. In certain embodiments, these terms include incorporating, formulating or otherwise including such agent into a composition that allows for sustained release of such agent in the desired application. The terms may contemplate any manner by which a therapeutic agent or other material is incorporated into a matrix, including, for example, distributed throughout the matrix, appended to the surface of the matrix (by intercalation or other binding interactions), encapsulated inside the matrix, etc. The term "co-incorporation" or "co-encapsulation" refers to the incorporation of a therapeutic agent or other material and at least one other therapeutic agent or other material in a subject composition.

In one aspect of this invention, a composition comprising a vitrigel composition as claimed in claim 1 and one or more biologically active agents may be prepared. The biologically active agent may vary widely with the intended purpose for the composition. The term active is art-recognized and refers to any moiety that is a biologically, physiologically, or pharmacologically active substance that acts locally or systemically in a subject. Examples of biologically active agents, that may be referred to as "drugs", are described in well-known literature references such as the Merck Index, the Physicians' Desk Reference, and The Pharmacological Basis of Therapeutics, and they include, without limitation, medicaments; vitamins; mineral supplements; substances used for the treatment, prevention, diagnosis, cure or mitigation of a disease or illness; substances which affect the structure or function of the body; or pro-drugs, which become biologically active or more active after they have been placed in a physiological environment. Various forms of a biologically active agent may be used which are capable of being released by the vitrigel composition, for example, into adjacent tissues or fluids upon administration to a subject. In some examples of the disclosure, a biologically active agent may be used to, for example, treat, ameliorate, inhibit, or prevent a disease or symptom, in conjunction with, for example, the eye.

Non-limiting examples of biologically active agents include the following: adrenergic blocking agents, anabolic agents, androgenic steroids, anti-allergenic materials, anti-cholinergics and sympathomimetics, anti-hypertensive agents, anti-infective agents, anti-inflammatory agents such as steroids, non-steroidal anti-inflammatory agents (NSAIDS), anti-pyretic and analgesic agents, antihistamines, biologicals, decongestants, diagnostic agents, estrogens, ion exchange resins, mitotics, mucolytic agents, growth factors, neuromuscular drugs, nutritional substances, peripheral vasodilators, progestational agents, prostaglandins, vitamins, antigenic materials, and prodrugs.

Examples of NSAIDS used in the compositions of the present invention can include mefenamic acid, aspirin, Diflunisal, Salsalate, Ibuprofen, Naproxen, Fenoprofen, Ketoprofen, Deacketoprofen, Flurbiprofen, Oxaprozin, Loxoprofen, Indomethacin, Sulindac, Etodolac, Ketorolac, Diclofenac, Nabumetone, Piroxicam, Meloxicam, Tenoxicam, Droxicam, Lornoxicam, Isoxicam, Meclofenamic acid, Flufenamic acid, Tolfenamic acid, elecoxib, Rofecoxib, Valdecoxib, Parecoxib, Lumiracoxib, Etoricoxib, Firocoxib, Sulphonanilides, Nimesulide, Niflumic acid, and Licofelone.

Various forms of the biologically active agents may be used. These include, without limitation, such forms as uncharged molecules, molecular complexes, salts, ethers, esters, amides, prodrug forms and the like, which are biologically activated when implanted, injected or otherwise placed into a subject.

The vitrigel compositions will be formulated, dosed and administered in a manner consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "therapeutically effective amount" of the vitrigel compositions to be administered will be governed by such considerations, and can be the minimum amount necessary to prevent, ameliorate or treat a disorder of interest. As used herein, the term "effective amount" is an equivalent phrase refers to the amount of a therapy (e.g., a prophylactic or therapeutic agent), which is sufficient to reduce the severity and/or duration of a disease, ameliorate one or more symptoms thereof, prevent the advancement of a disease or cause regression of a disease, or which is sufficient to result in the prevention of the development, recurrence, onset, or progression of a disease or one or more symptoms thereof, or enhance or improve the prophylactic and/or therapeutic effect(s) of another therapy (e.g., another therapeutic agent) useful for treating a disease.

In one example of the disclosure, the repair of damaged tissue may be carried out within the context of any standard surgical process allowing access to and repair of the tissue, including open surgery and laparoscopic techniques. Once the damaged tissue is accessed, a vitrigel composition of the invention is placed in contact with the damaged tissue along with any surgically acceptable patch or implant, if needed.

Disclosed, but not claimed, is a method for making a vitrified matrix gel having a first component and a second component, wherein the first component comprises collagen, and wherein the second component comprises cyclodextrin, comprising: a) obtaining an aqueous solution of collagen; b) obtaining an aqueous solution of cyclodextrin; c) combining the solutions of a) and b); and d) dehydrating the combined solution of c) for a period of time sufficient to allow vitrification of the solution.

As used herein, the aqueous solution of collagen is any collagen solution dissolved in a suitable buffer. The concentration of the collagen is variable, however solutions of collagen with a concentration in a range of 1 mg/ml to about 10 mg/ml can be used with the methods disclosed herein. In an example of the disclosure, the concentration of collagen in aqueous solution is about 5 mg/ml.

As used herein, the aqueous solution of cyclodextrin is any cyclodextrin solution dissolved in a suitable buffer. The concentration of the cyclodextrin is variable, however solutions of cyclodextrin with a concentration in a range of 2.5 mg/ml to about 10 mg/ml can be used with the methods disclosed herein.

In accordance with the disclosed methods the dehydration and vitrification of the composition of the present invention comprises drying the solution comprising the collagen solution and cyclodextrin at a temperature of about 5 to 40 °C, at a relative humidity of between about 30 to 50%, and for a time of about 3 days to about 28 days. In an example of the disclosure, vitrification of the composition of the present invention comprises heating the solution comprising the collagen solution and cyclodextrin at a temperature of 39 °C for about 7 days. In another example of the disclosure, vitrification of the composition of the present invention comprises heating the solution comprising the collagen solution and cyclodextrin at a temperature of 39 °C for about 14 days.

The term, "carrier," refers to a diluent, adjuvant, excipient or vehicle with which the therapeutic is supplied with the vitrigel composition of the present invention. Such physiological carriers can be sterile liquids, such as water and oils, including those of petroleum, animal, vegetable or synthetic origin, such as peanut oil, soybean oil, mineral oil, sesame oil and the like. Water is a suitable carrier when the pharmaceutical composition is administered intravenously. Saline solutions and aqueous dextrose and glycerol solutions also can be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol and the like. The composition, if desired, can also contain minor amounts of wetting or emulsifying agents, or pH buffering agents.

Buffers, acids and bases may be incorporated in the compositions to adjust pH. Agents to increase the diffusion distance of agents released from the composition may also be included.

Buffering agents help to maintain the pH in the range which approximates physiological conditions. Buffers are preferably present at a concentration ranging from about 2 mM to about 50 mM. Suitable buffering agents for use with the instant invention include both organic and inorganic acids, and salts thereof, such as citrate buffers (e.g., monosodium citrate-disodium citrate mixture, citric acid-trisodium citrate mixture, citric acid-monosodium citrate mixture etc.), succinate buffers (e.g., succinic acid monosodium succinate mixture, succinic acid-sodium hydroxide mixture, succinic acid-disodium succinate mixture etc.), tartrate buffers (e.g., tartaric acid-sodium tartrate mixture, tartaric acid-potassium tartrate mixture, tartaric acid-sodium hydroxide mixture etc.), fumarate buffers (e.g., fumaric acid-monosodium fumarate mixture, fumaric acid-disodium fumarate mixture, monosodium fumarate-disodium fumarate mixture etc.), gluconate buffers (e.g., gluconic acid-sodium glyconate mixture, gluconic acid-sodium hydroxide mixture, gluconic acid-potassium gluconate mixture etc.), oxalate buffers (e.g., oxalic acid-sodium oxalate mixture, oxalic acid-sodium hydroxide mixture, oxalic acid-potassium oxalate mixture etc.), lactate buffers (e.g., lactic acid-sodium lactate mixture, lactic acid-sodium hydroxide mixture, lactic acid-potassium lactate mixture etc.) and acetate buffers (e.g., acetic acid-sodium acetate mixture, acetic acid-sodium hydroxide mixture etc.). Phosphate buffers, carbonate buffers, histidine buffers, trimethylamine salts, such as Tris, HEPES and other such known buffers can be used.

Preservatives may be added to retard microbial growth, and may be added in amounts ranging from 0.2%-1 % (w/v). Suitable preservatives for use with the present invention include phenol, benzyl alcohol, m-cresol, octadecyldimethylbenzyl ammonium chloride, benzyaconium halides (e.g., chloride, bromide and iodide), hexamethonium chloride, alkyl parabens, such as, methyl or propyl paraben, catechol, resorcinol, cyclohexanol and 3-pentanol.

Isotonicifiers are present to ensure physiological isotonicity of liquid compositions of the instant invention and include polhydric sugar alcohols, preferably trihydric or higher sugar alcohols, such as glycerin, erythritol, arabitol, xylitol, sorbitol and mannitol. Polyhydric alcohols can be present in an amount of between about 0.1 % to about 25%, by weight, preferably 1% to 5% taking into account the relative amounts of the other ingredients.

The formulations to be used for in vivo administration must be sterile. That can be accomplished, for example, by filtration through sterile filtration membranes. For example, the formulations of the present invention may be sterilized by filtration.

In accordance with one or more embodiments, there is provided ophthalmic formulations comprising the compositions of the present invention, wherein the formulation is suitable for administration to the eye of a subject. The ophthalmic formulation may have a pH between 5.5 and 7. In some embodiments the ophthalmic formulation is an aqueous formulation. In some embodiments the ophthalmic formulation is in the form of a single dose unit. In some embodiments the ophthalmic formulation does not comprise a preservative. The ophthalmic formulation may further comprise one or more additional therapeutic agents, such as antioxidants. The ophthalmic formulation may further comprise one or more tear substitutes. In some embodiments, at least one of the tear substitutes contains an ophthalmic lubricant (e.g., hydroxypropylmethylcellulose).

A variety of tear substitutes are known in the art and include, but are not limited to: monomeric polyols, such as, glycerol, propylene glycol, and ethylene glycol; polymeric polyols such as polyethylene glycol; cellulose esters such hydroxypropylmethyl cellulose, carboxy methylcellulose sodium and hydroxy propylcellulose; dextrans such as dextran 70; water soluble proteins such as gelatin; vinyl polymers, such as polyvinyl alcohol, polyvinylpyrrolidone, and povidone; and carbomers, such as carbomer 934P, carbomer 941, carbomer 940 and carbomer 974P. Many such tear substitutes are commercially available, which include, but are not limited to cellulose esters such as Bion Tears®, Celluvisc®, Genteal®, OccuCoat®, Refresh®, Teargen II®, Tears Naturale®, Tears Natural II®, Tears Naturale Free®, and TheraTears®; and polyvinyl alcohols such as Akwa Tears®, HypoTears®, Moisture Eyes®, Murine Lubricating®, and Visine Tears®. Tear substitutes may also be comprised of paraffins, such as the commercially available Lacri-Lube® ointments. Other commercially available ointments that are used as tear substitutes include Lubrifresh PM®, Moisture Eyes PM® and Refresh PM®. Preservatives and other additives may also be present such as, for example, antimicrobials, antioxidants, chelating agents, and inert gases and the like.

### EXAMPLES

Type I collagen-based membranes incorporated with different cyclodextrins were prepared following a three-stage sequence: gelation, vitrification and rehydration. Three types of cyclodextrins were tested and compared, i.e. α-CD, β-CD and γ-CD. The examples in which cyclodextrins do not comprise a plurality of hydroxyl groups chemically substituted with a different functional group or moiety wherein the different functional group or moiety is a hydrophilic group are reference examples.

Preparation of CD solutions: All solutions with various CDs (e.g., α, β and functional groups, COOH, t-butyl, SH, NH₂) were prepared in a procedure as described here: For example, a β-CD solution (2.5 mg/mL) in dH₂O with 20 nM HEPES (1 mL HEPES, 49 mL H₂O and 125 mg β-CD in a 50 ml falcon tube) was added and vortexed thoroughly to dissolve completely. Subsequently, the pH of this solution was increased 11 using a sodium hydroxide (2N) solution, filtered through a 0.22 µm syringe filter and stored at 1-4 °C.

Preparation of β-CD Collagen gel: Various gels of collagen and CDs were prepared in a procedure as described here: For example, an equal volume of a β-CD solution (2.5 mg/mL and as prepared above) and a collagen acetic acid solution (5 mg/mL) were mixed together and kept at 4 °C or lower throughout the preparation. Special care was taken to prevent air bubbles formation due to mixing. Then after, the mixture was transferred into gel molds and kept at 37 °C 100% RH incubator for 2 hours.

Vitrification and post-processing of β-CD Collagen: Following 2 hour incubation at 37 °C, gels were transferred to 5 °C (40% RH) vitrification chamber for 18 hours. Next, gels were transferred to 40 °C vitrification chamber (40% RH) for one week. After a week, dehydrated β-CD Collagen membranes (vitrigels) were rehydrated in dH₂O for 30 minutes. If needed, vitrigels were crosslinked using 0.1% EDAC and 0.1% NHS in dH₂O for 30 min in 37 °C, washed vitrigels using dH₂O at least five times to remove traces of EDAC and NHS. The hydrophilic functional groups induce lamella structures as shown in Fig. IB.

Two collagen membranes, i.e. collagen vitrigel and crosslinked vitrigel, were prepared as controls following the procedures as previously described (Biomaterials 34 (2013) 9365-9372). Compared to normal vitrigel, the crosslinked vitrigel was fabricated with additional 0.6% 1-ethyl-3-(3-dimethylaminopropyl) carbodiimide (EDC) and 0.6% N-hydroxysuccinimide (NHS) in the collagen media mixture before the gelation process. The specific interactions between collagen and CD were evaluated using differential scanning calorimetry (DSC, Perkin Elmer, Waltham, MA). Absorbance of CD-col membranes was measured using a Synergy 2 microplate reader (BioTek, Winooski, VT). Membrane suturability was tested with a 10-0 nylon suture using an Electroforce 3200 testing instrument (Bose, Eden Prairie, MN).

Keratocytes were isolated from full-thickness corneas using a sequential collagenase (Type 2, Worthington Biochemical Corp., Lakewood, NJ) digestion. The digested cells were collected and centrifuged at 1400 rpm for 10 minutes. The cell pellet was resuspended and cultured on general tissue culture plates (TCP) or CV-covered plates at 37 °C and 5% CO₂. Either serum-free or serum based culture medium was used. The serum-free medium consisted of DMEM/F-12, 1% of 10 U/mL penicillin-streptomycin, and 0.5% of 1.25 mg/mL amphotericin B (Life Technologies, Carlsbad, CA). The serum-based medium contained DMEM/F-12, 10% FBS, 1% of 10 U/mL penicillin-streptomycin, and 0.5% of 1.25 mg/mL amphotericin B. The cells were plated at a concentration of 5000 cells/cm² using the serum-free medium, while at a concentration of 1000 cells/cm² using the serum-based medium. Before imaging and gene expression analyses, keratocytes were cultured over 3 wk in serum-free medium or 6 d in serum-based medium. Keratocyte morphologies were examined by staining with the LIVE/DEAD® Viability/Cytotoxicity Kit (Life Technologies). Indomethacin was encapsulated in the vitrigels by soaking the vitrified membranes in 0.1% indomethacin eye drops for a period of time. The elution of indomethacin from the vitrigel was measured using high-performance liquid chromatography (HPLC). The release solution was tested using a mobile phase of acetonitrile:water of 51:49 (v/v), a C18 column and a UV/VIS detector set at 318 nm.

### EXAMPLE 1

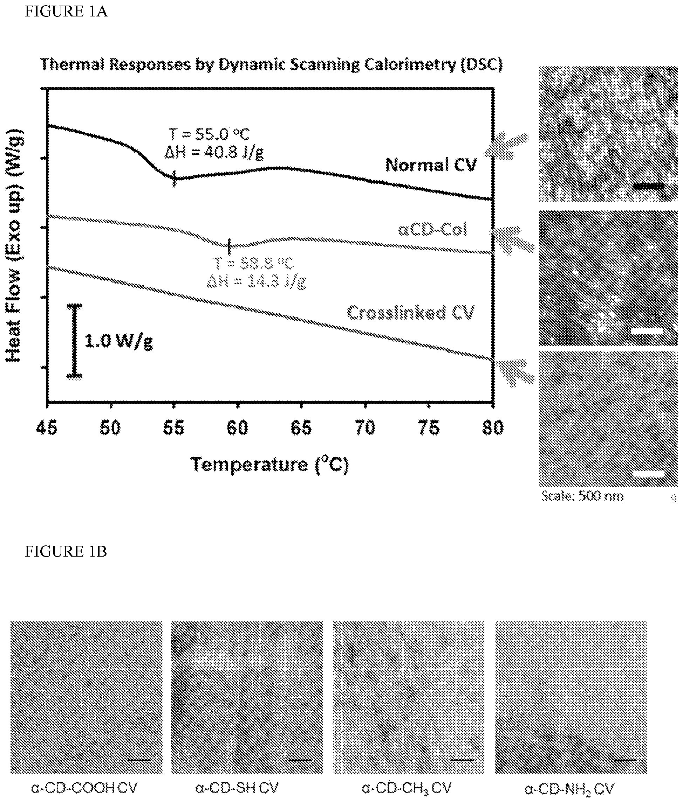

All three cyclodextrins, especially α-CD, exhibited strong interactions with type I collagen triple helices, leading to formation of transparent and mechanically strong CD-col membranes. As shown in Figure 1A, normal vitrigel exhibited a large and broad endothermic peak at 55 °C in the heat flow, which indicates that the collagen membrane underwent a thermal denaturation with an enthalpy of 40.8 J/g. In contrast, no discernible peak was found in the control sample of crosslinked vitrigel, suggesting a denatured feature of the collagen membrane due to the crosslinking reaction. Compared to normal vitrigel, the addition of a-CD in collagen membrane led to an increased denaturation temperature, indicating an enhanced thermal stability. Only a single narrower peak was observed in α-CD-col, which means that the matrix had a homogeneous structure. If we assume that all of the enthalpy comes from the thermal transition of collagen triple helices, the denaturation enthalpy of a-CD-col was 70.1% of that from normal vitrigel, suggesting a reduced collagen fibrogenesis. Similar results were also observed in β-CD-col and γ-CD-col.

### EXAMPLE 2

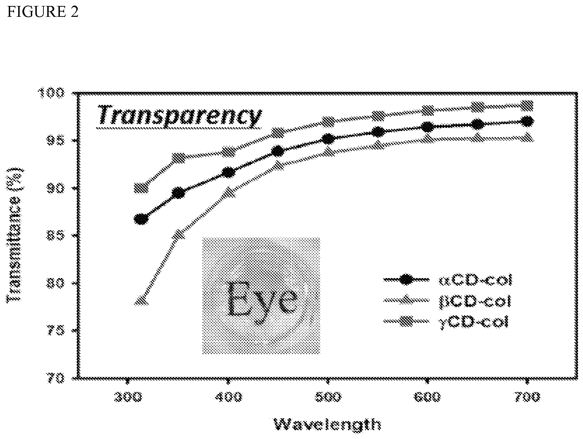

Compared to conventional collagen membrane, the CD-col membranes showed greatly enhanced transparency (Fig. 2), which can be explained by the reduced collagen fibrogenesis.

Type I collagen-CD membranes were developed with optimized optical and mechanical properties for corneal regeneration. CDs represent a ring of six to eight glucose molecules with an inner hydrophobic core and an outer hydrophilic ring. All three CDs, especially α-CD, exhibited strong interactions with collagen triple helices, leading to formation of mechanically strong collagen-CD membranes. The collagen-CD membranes showed significantly higher transparency than conventional collagen membranes, which can be explained by the greatly reduced the collagen fibril diameter in collagen-CD membranes (∼20 nm) compared to conventional collagen membranes (∼80 nm). Furthermore, unlike conventional collagen membrane exhibiting a random fibrillar organization, the collagen-CD membranes demonstrated aligned fibrils in some regions probably due to reorganization of collagen triple helices by CD. In addition, it was found that the collagen-CD membrane could absorb the ophthalmic drug, indomethacin, from eye drops and then slowly release the drug up to five hours.

The incorporation of cyclodextrin in type I collagen membranes regulated collagen fibrillogenesis and alignment and improved optical, mechanical and drug release properties in membranes.

### EXAMPLE 3

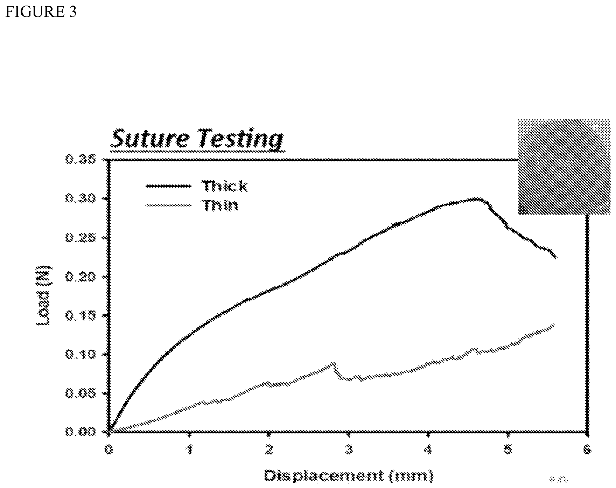

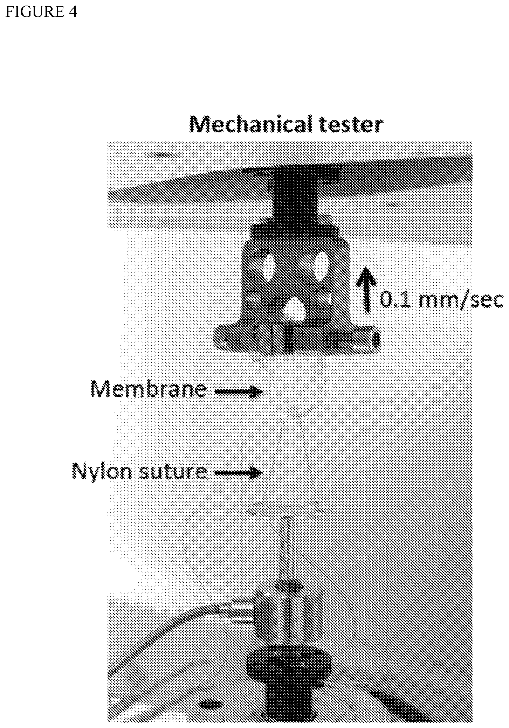

The inventive compositions demonstrated superior mechanical properties. When their thickness was comparable to that of human cornea (i.e. ∼500 µm), they became strong enough for suture. As shown in Figure 3, a nylon suture was pulling through a hole in α-CD-col membrane with a thickness of 520 µm. Under the stress of suture, the hole in the thick membrane was only stretched, instead of tearing through the membrane as observed in the thin one with a thickness of 170 µm (Fig. 4).

### EXAMPLE 4

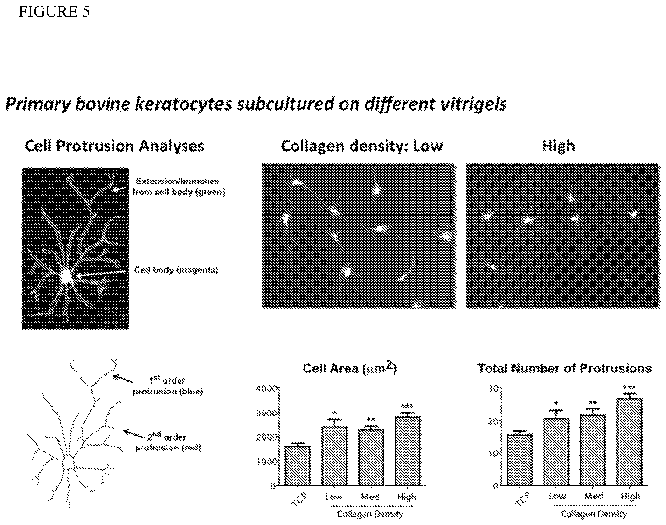

Collagen nanoarchitecture defines cell response. Primary cultures of bovine keratocytes were cultured on vitrigel compositions having low and high collagen density. As shown in Figure 5, collagen density of the compositions of the present invention allowed the keratocytes to have greater protrusion area in culture when compared with normal vitrigel controls. In addition, the total number of cell protrusions of the keratocytes were significantly increased as a function of the collagen density of the inventive vitrigel compositions were increased (Fig. 5).

### EXAMPLE 5

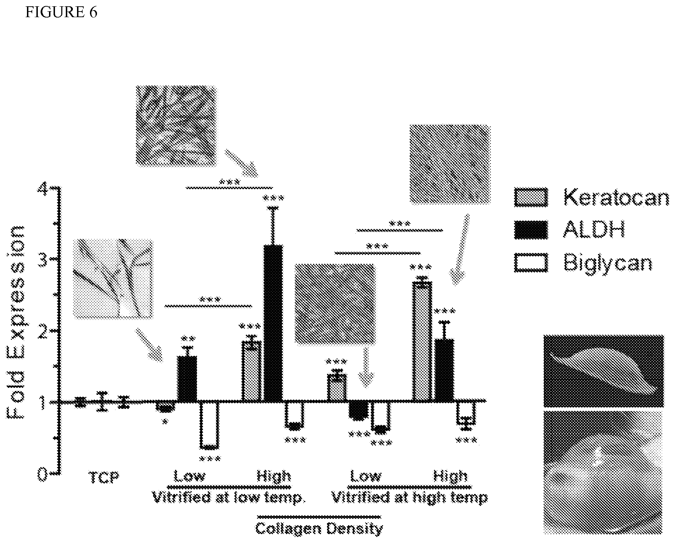

Keratocyte gene expression is dependent on fibril architecture. Keratocytes were cultured on control vitrigels or with the inventive vitrigel compositions where the vitrigels were dehydrated at 5 °C or 39 °C temperatures. After growth for 6 days in serum-based medium, the cells were harvested and analyzed for gene expression of keratocan, aldehyde dehydrogenase (ALDH) and biglycan. The expression of these genes was analyzed by isolating total RNA from cultured keratocytes using TRIzol reagent (Life Technologies), reverse transcribing into cDNA using SuperScript II First Strand Synthesis Kit (Life Technologies) and then testing the cDNA using real-time PCR reactions on a StepOnePlus Real-Time PCR System (Applied Biosystems®, Life Technologies). As shown in Fig. 6, when compared with controls, the gene expression of keratocan and ALDH was greatly increased when the cells were grown on the vitrigel compositions dehydrated at high temperature. Expression of biglycan was reduced in the vitrigel compositions when compared to controls at both low and high temperatures.

### EXAMPLE 6

The vitrigel compostions of the present invention can be used to deliver biologically active agents. The vitrigel compositions were prepare as above, and a solution of a commercially available eye drop formulation of 0.1% indomethacin in ethanol was added to the composition for either 10 minutes using two drops or overnight soaking in 1 mL eye drop after hydrating the vitrigels, and the release kinetics were tested using HPLC. It was found that the vitrigel composition released the indomethacin from the vitrigel composition over a 5 hour period.

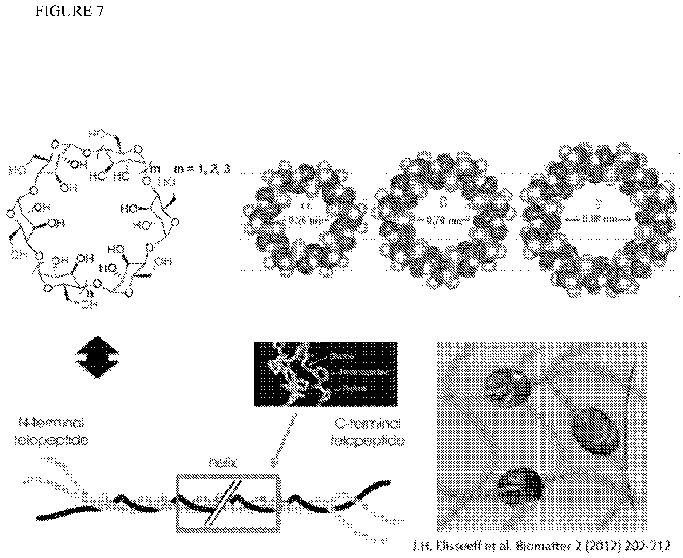

Type I collagen-CD compositions were developed with optimized optical and mechanical properties for corneal regeneration. While not being limited to any particular theory, these properties are probably due to regulated collagen fibrillogenesis in the cyclodextrin-incorporated collagen compositions (Fig. 7). These compositions hold a great potential to be used as therapeutic eye patch for corneal repair and treatments. The compositions and methods disclosed herein may also be useful for regeneration of other connective tissues derived from fibril-forming collagens, such as cartilage, skin and blood vessel.

### EXAMPLE 7

The effect of the interaction of collagen with cyclodextrins on collagen fibrillar organization was studied. Collagen type I in an acidic solution is combined with cyclodextrins solutions of varying compositions. This solution can then be buffered to raise the pH to form gels. These gels are then vitrified over a 1 week period, following which the dry sheets of CD-Col were rehydrated. A variety of sample characterizations were carried out to match the requirements of such an implant in the clinic.

Material was prepared as follows: hydrogel was formed using collagen type I and cyclodextrin solutions in appropriate buffer. The vitrification process was at controlled temperature and relative humidity over a1 week period. Cyclodextrin collagen vitrigel implants were rehydrated.

Samples required the following clinical properties/tests: light transmittance; transmission electron microscopy; mechanical testing; in vitro biocompatibility studies; preliminary in vivo studies.

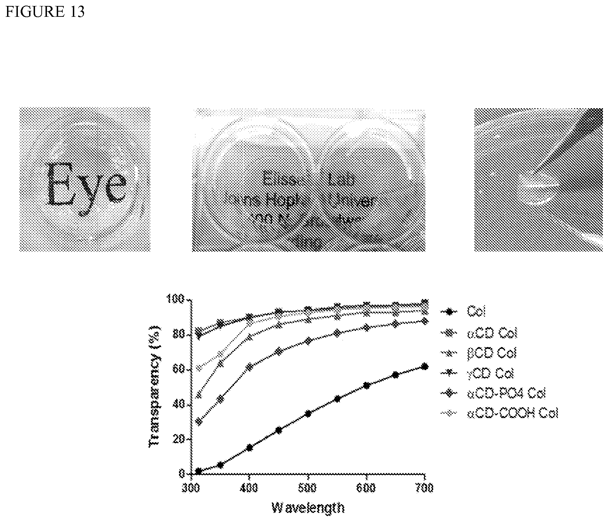

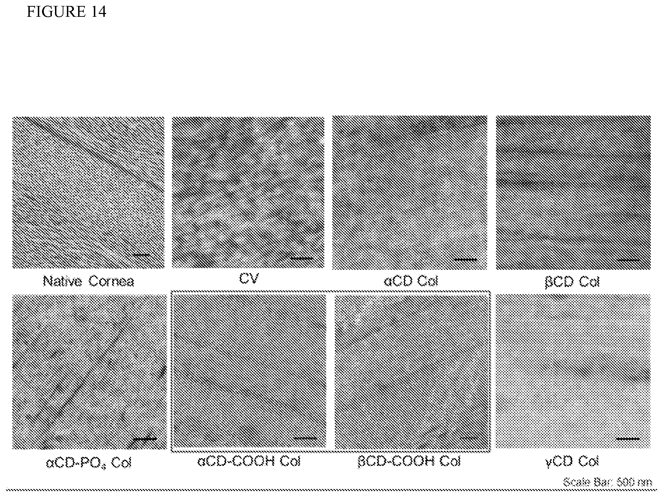

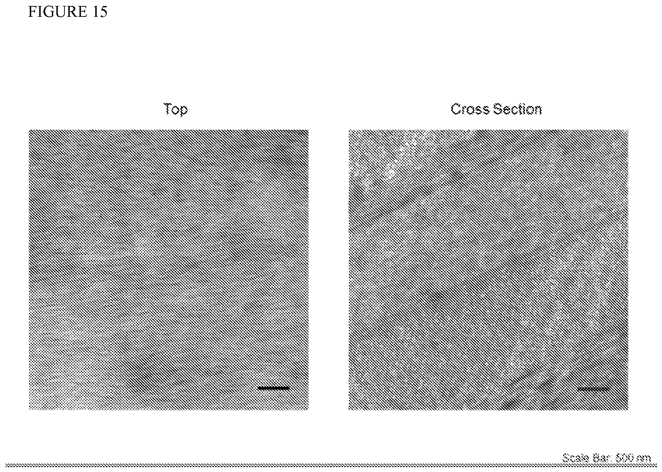

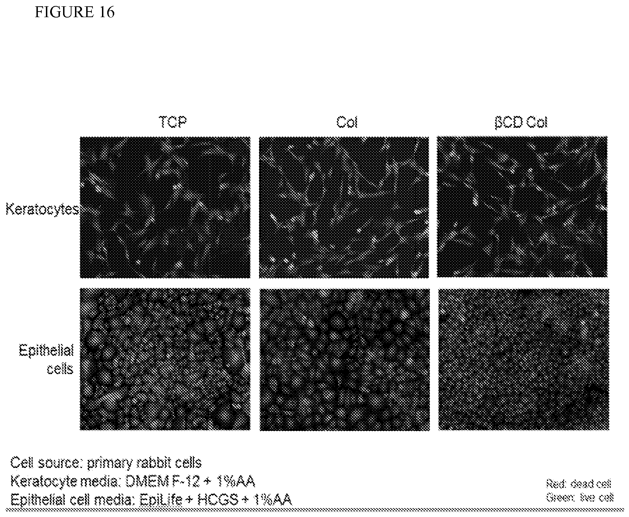

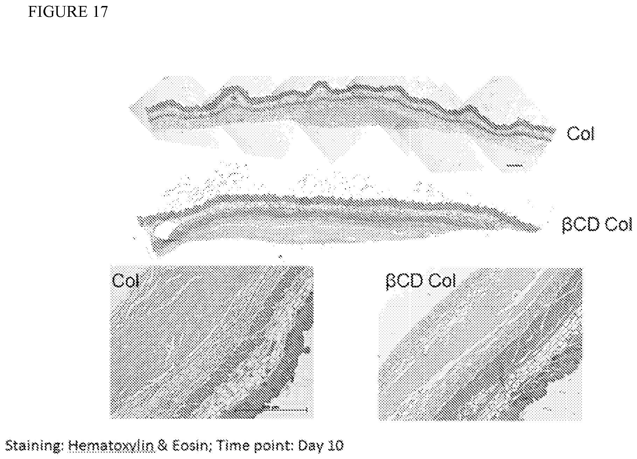

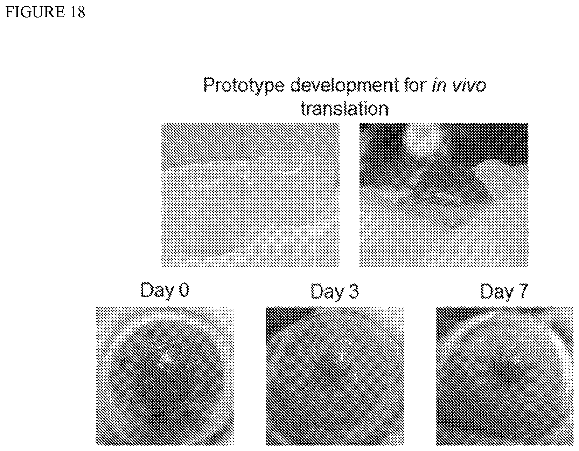

These prepared cyclodextrin/collagen materials, even at thickness of 500 microns were clear, especially when compared to the traditional vitrigels without cyclodextrin. Further, it was observed that depending on the type and functional group of the cyclodextrin, variations in transparency were seen. Most of these implants showed transparencies comparable to human corneas, namely above 90% in the visible light range. Interestingly, it was also observed that these thick sheets could, with sufficient force, be separated layer by layer into thinner sheets (See, Fig. 13). This prompted us to visualize these in TEM, to figure out where these layers were coming from. A variety of ultrastructures were seen. Particularly fascinating was the CDs with COOH functional groups, which exhibited self assembled lamellae similar to the lamellae of native corneal stroma (See, Fig. 14). Not only did these self assemble into lamellae, as can be seen in the cross section, but collagen in each lamella were demonstrated fiber alignment and fibers in adjacent lamellae were running orthogonal to each other (See, Fig. 15). Mechanical tests were run to determine the strengths of the implants with individual CD components and also saw a wide distinction between the types of CD. It was interesting to note that β-CD Collagen implants had the highest Young's modulus, and all implants with CD demonstrate very high strain at break - which implies that these materials were highly stretchable, a property that was seen directly translated to fracture resistance, or in this case, suturability. To confirm the implants were biocompatible, in vitro cell cultures of both Keratocytes and Epithelial cells with the implants were examined. As can be seen, the implants had no issues growing on these surfaces (Fig. 16). Further, though somewhat unconventional, biocompatibility subcutaneously was also tested. If subcutaneous implants did not cause any adverse immune reactions, it can be said that most likely, these implants would not have a negative effect on the immune privileged cornea (Fig. 17). In addition, these implants were developed in special molds to match the curvature of the eye. As can be seen on the right, the implant sits well on the corneal surface (Fig. 18).

A pilot study was undertaken by implanting these corneas into the rabbit eyes. Regular interrupt sutures were able to hold the material well (Fig. 18). It was concluded that CD molecules act as artificial chaperones, which direct the assembly of collagen fibrils mimicking organization of native cornea. CD Collagen implants are biocompatible and have cornea-mimetic properties. Ultrastructural and macroscopic properties modulated by CD functionalization. CD Collagen implants show potential as corneal substitutes due to the high transparency, ease of suturability and biomimetic ultrastructure.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

## Claims

1. A composition comprising aligned fibrils of a vitrified matrix gel comprising collagen and cyclodextrin (CD), wherein the composition comprises α-CD, β-CD and/or γ-CD, **characterised in that** the composition is multilayered and the α-CD, β-CD and/or γ-CD comprise a plurality of hydroxyl groups chemically substituted with a different functional group or moiety wherein the different functional group or moiety is a hydrophilic group.

2. The composition of claim 1, wherein the composition comprises Type I, Type II, Type III and/or Type IV collagen.

3. The composition of claim 1 or 2, wherein the hydrophilic group is selected from the groups consisting of COOH, SH and NH₂.

4. The composition of claim 3, wherein the hydrophilic group is COOH.

5. The composition of any preceding claim, wherein the composition comprises Type I collagen.

6. The composition of any preceding claim, wherein the composition comprises α-cyclodextrin.

7. The composition of any preceding claim, wherein a substantial portion of the collagen content of the composition is Type 1 collagen and/or wherein a substantial portion of the cyclodextrin content of the composition is α-CD.

8. The composition of any one of the preceding claims, wherein the composition further comprises at least one biologically active agent.

9. The composition of any one of the preceding claims, wherein the thickness of the layered composition is between 10 and 500 microns.

10. The composition of any preceding claim, additionally including a surface coating to enhance biological action.

11. The composition of any preceding claim, wherein the composition is formed as a corneal replacement, patch or graft.

12. The composition of claim 11, wherein the composition is hydrated prior to use and has an optical transparency of above 90% at 550 nm.

13. The composition of any one of claims 1 to 12 for use as a medicament.

14. The composition of any one of claims 1 to 12 for use as a matrix for repair of a tissue of a mammal.

15. The composition of any one of claims 1 to 12 for use as a matrix for repair of a cornea of an eye of a mammal.

## Patentansprüche

1. Eine Zusammensetzung, beinhaltend ausgerichtete Fibrillen eines verglasten Matrixgels, beinhaltend Kollagen und Cyclodextrin (CD), wobei die Zusammensetzung α-CD, β-CD und/oder y-CD beinhaltet, **gekennzeichnet dadurch, dass** die Zusammensetzung mehrschichtig ist und das α-CD, β-CD und/oder y-CD eine Vielzahl von Hydroxylgruppen, die chemisch mit einer unterschiedlichen funktionellen Gruppe oder Einheit substituiert sind, beinhalten, wobei die andere funktionelle Gruppe oder Einheit eine hydrophile Gruppe ist.

2. Zusammensetzung gemäß Anspruch 1, wobei die Zusammensetzung Kollagen vom Typ I, Typ II, Typ III und/oder Typ IV beinhaltet.

3. Zusammensetzung gemäß Anspruch 1 oder 2, wobei die hydrophile Gruppe ausgewählt ist aus den Gruppen, bestehend aus COOH, SH und NH₂.

4. Zusammensetzung gemäß Anspruch 3, wobei die hydrophile Gruppe COOH ist.

5. Zusammensetzung gemäß einem der vorhergehenden Ansprüche, wobei die Zusammensetzung Kollagen vom Typ I beinhaltet.

6. Zusammensetzung gemäß einem der vorhergehenden Ansprüche, wobei die Zusammensetzung α-Cydodextrin beinhaltet.

7. Zusammensetzung gemäß einem der vorhergehenden Ansprüche, wobei ein wesentlicher Teil des Kollagengehalts der Zusammensetzung Kollagen vom Typ 1 ist und/oder wobei ein wesentlicher Teil des Cyclodextringehalts der Zusammensetzung α-CD ist.

8. Zusammensetzung gemäß einem der vorhergehenden Ansprüche, wobei die Zusammensetzung ferner mindestens einen biologischen Wirkstoff beinhaltet.

9. Zusammensetzung gemäß einem der vorhergehenden Ansprüche, wobei die Dicke der schichtigen Zusammensetzung zwischen 10 und 500 Mikrometer liegt.

10. Zusammensetzung gemäß einem der vorhergehenden Ansprüche, zusätzlich umfassend eine Oberflächenbeschichtung zur Verbesserung der biologischen Wirkung.

11. Zusammensetzung gemäß einem der vorhergehenden Ansprüche, wobei die Zusammensetzung als Hornhautersatz, -patch oder-transplantat gebildet ist.

12. Zusammensetzung gemäß Anspruch 11, wobei die Zusammensetzung vor der Verwendung hydratisiert wird und eine optische Transparenz von über 90 % bei 550 nm aufweist.

13. Zusammensetzung gemäß einem der Ansprüche 1 bis 12 zur Verwendung als Medikament.

14. Zusammensetzung gemäß einem der Ansprüche 1 bis 12 zur Verwendung als Matrix zur Reparatur eines Gewebes eines Säugetiers.

15. Zusammensetzung gemäß einem der Ansprüche 1 bis 12 zur Verwendung als Matrix zur Reparatur einer Hornhaut eines Auges eines Säugetiers.

## Revendications

1. Une composition comprenant des fibrilles alignées d'un gel de matrice vitrifié comprenant du collagène et de la cyclodextrine (CD), la composition comprenant de l'a-CD, de la β-CD et/ou de la γ-CD, **caractérisée en ce que** la composition est multicouche et l'a-CD, la β-CD et/ou la γ-CD comprennent une pluralité de groupes hydroxyle chimiquement substitués par un groupe fonctionnel ou une partie différent(e), le groupe fonctionnel ou la partie différent(e) étant un groupe hydrophile.

2. La composition de la revendication 1, la composition comprenant du collagène de type I, de type II, de type III et/ou de type IV.

3. La composition de la revendication 1 ou de la revendication 2, dans laquelle le groupe hydrophile est sélectionné parmi les groupes consistant en COOH, SH et NH₂.

4. La composition de la revendication 3, dans laquelle le groupe hydrophile est COOH.

5. La composition de n'importe quelle revendication précédente, la composition comprenant du collagène de type I.

6. La composition de n'importe quelle revendication précédente, la composition comprenant de l'a-cyclodextrine.

7. La composition de n'importe quelle revendication précédente, dans laquelle une portion substantielle du collagène contenu dans la composition est du collagène de type 1 et/ou dans laquelle une portion substantielle de la cyclodextrine contenue dans la composition est de l'α-CD.

8. La composition de n'importe laquelle des revendications précédentes, la composition comprenant en sus au moins un agent biologiquement actif.

9. La composition de n'importe laquelle des revendications précédentes, dans laquelle l'épaisseur de la composition en couches est comprise entre 10 et 500 microns.

10. La composition de n'importe quelle revendication précédente, incluant de surcroît un revêtement de surface afin de renforcer l'action biologique.

11. La composition de n'importe quelle revendication précédente, la composition étant formée en tant qu'élément de remplacement, patch ou greffon cornéen.

12. La composition de la revendication 11, la composition étant hydratée préalablement à une utilisation et ayant une transparence optique de plus de 90 % à 550 nm.

13. La composition de n'importe laquelle des revendications 1 à 12 pour une utilisation en tant que médicament.

14. La composition de n'importe laquelle des revendications 1 à 12 pour une utilisation en tant que matrice pour la réparation d'un tissu d'un mammifère.

15. La composition de n'importe laquelle des revendications 1 à 12 pour une utilisation en tant que matrice pour la réparation d'une cornée d'un œil d'un mammifère.
